# NEUE EUROPÄISCHE PATENTSCHRIFT

(11) **EP 2 691 023 B2**
(45) Veröffentlichungstag und Bekanntmachung des Hinweises auf die Entscheidung über den Einspruch: **26.12.2018**
(45) Hinweis auf die Patenterteilung: 07.01.2015
(21) Anmeldenummer: 12713070.6
(22) Anmeldetag: 28.03.2012
(51) Int. Cl.: A61B 5/151, A61B 5/15

(54) **LANZETTENMAGAZIN FÜR STECHHILFEVORRICHTUNGEN**
LANCET MAGAZINE FOR PUNCTURING AIDS
MAGASIN DE LANCETTES

(30) Priorität: 30.03.2011 DE 102011015656
(43) Veröffentlichungstag der Anmeldung: 05.02.2014
(73) Patentinhaber: Gerresheimer Regensburg GmbH, 93047 Regensburg (DE)
(72) Erfinder: VOLKMUTH, Julia, 93142 Maxhütte-Haidhof (DE); STREHL, Michael-Martin, 92536 Pfreimd (DE)
(74) Vertreter: Hannke, Christian
(86) Internationale Anmeldenummer: PCT/EP2012/055544
(87) Internationale Veröffentlichungsnummer: WO 2012/130899

(56) Entgegenhaltungen:
- EP-A1- 1 459 683
- EP-A1- 2 218 399
- EP-A2- 1 190 674
- EP-A2- 1 190 674
- WO-A2-2008/140464
- DE-A1- 10 022 720
- DE-A1- 10 053 974
- US-A1- 2008 109 024

## Beschreibung

Die Erfindung betrifft eine Vorrichtung zum Bevorraten und Bereitstellen von Lanzetten zur Gewinnung von Körperflüssigkeiten, wobei die Vorrichtung ein Lanzettenmagazin mit einer Lanzettenbevorratungseinheit für eine Mehrzahl von nadelförmigen Lanzetten und eine Antriebseinheit zur Ausführung eines Stechvorganges einer Lanzette umfasst und eine Rückholeinheit vorhanden ist, die nach der Ausführung des Stechvorganges die Lanzette von der Stechposition in ihre Ruheposition zurückbewegt.

Derartige Vorrichtungen zum Bevorraten und Bereitstellen von Lanzetten sind bevorzugt für die Untersuchung von Proben von Körperflüssigkeiten, insbesondere von Blutproben, eingesetzt. Diese Untersuchung ermöglicht in der klinischen Diagnostik das frühzeitige und zuverlässige Erkennen von pathologischen Zuständen sowie die gezielte und fundierte Kontrolle von Körperzuständen. Hierbei setzt die medizinische Blutdiagnostik stets die Gewinnung einer Blutprobe des zu untersuchenden Individuums voraus. Während in Kliniken und bei niedergelassenen Ärzten oftmals durch eine Venenpunktion mehrere Milliliter Blut einer zu untersuchenden Person für die Analyse gefunden werden, um damit eine Vielzahl von Labortests durchführen zu lassen, reichen für eine einzelne Analyse, gezielt auf einen Parameter gerichtet, heutzutage oftmals wenige Mikroliter Blut, oder noch weniger aus. Es genügt hierfür, zur Blutgewinnung durch die Haut, zum Beispiel in die Fingerbeere oder das Ohrläppchen der zu untersuchenden Person mithilfe einer sterilen, scharfen Lanzette zu stoßen, um so einige wenige Mikroliter Blut oder sogar Blutmengen im Nanoliterbereich für die Analyse zu gewinnen. Vorrangig eignet sich diese Methode, wenn die Analyse der Blutprobe unmittelbar nach der Blutgewinnung durchgeführt werden kann.

Insbesondere, wenn medizinische Laien einfache Analysen des Blutes selbst durchführen, und für die regelmäßige, mehrmals tägliche durchzuführende Blutgewinnung durch Diabetiker für die Kontrolle der Blutglukosekonzentration werden Lanzetten und dazu passende Blutentnahmegeräte, Blutlanzettenvorrichtungen oder Stechhilfen angeboten, die eine möglichst schmerzarme und reproduzierbare Blutgewinnung ermöglichen. Derartige Lanzetten und Stechhilfen sind beispielsweise in EP A 0 5650970 gezeigt.

Bei den derzeit kommerziell verfügbaren Systemen erfolgt die Bereitstellung der Lanzetten für die Verwendung von Stechhilfen in loser Form oder es werden sogenannte Einmal-Stechhilfen verwendet, die eine einzige Lanzette aufweisen und anschließend weggeworfen oder zumindest gereinigt werden. Der Benutzer entnimmt für die Bereitstellung der Lanzetten in loser Form manuell vor jedem Stechvorgang eine Lanzette aus einer Verpackung, in der eine Vielzahl von Lanzetten enthalten ist. Anschließend wird die Stechhilfe für die Aufnahme der Lanzette vorbereitet, wobei der Lanzettenhalter der Stechhilfe freigelegt wird. Die aus der Verpackung entnommene Lanzette wird manuell in den Lanzettenhalter der Stechhilfe eingeführt und dort fixiert. Dann muss die Schutzhülle von der Lanzette manuell abgenommen werden. Anschließend wird die Stechhilfe mit ihrer Kappe wieder verschlossen. Die Kappe sorgt dafür, dass die Lanzette von außen nicht mehr zugänglich ist. Sie besitzt meist eine Öffnung, durch welche die Lanzettenspitze beim eigentlichen Stechvorgang austreten kann. Schließlich wird die Stechhilfe gespannt und steht für den Stechvorgang zur Gewinnung von Blut zur Verfügung.

Die Vielzahl der manuellen Bedienschritte bei herkömmlichen Lanzettensystemen wird vom Benutzer als nachteilig empfunden und ist vor allem bei eingeschränkter Wahrnehmung im Zustand einer Hypoglykämie problematisch. Zudem wird der Benutzer nicht daran gehindert, eine einmal eingelegte Lanzette mehrfach zum Stechen und Blutgewinnen zu verwenden, was dazu führt, dass die hygienischen Aspekte vernachlässigt werden.

Aus US 5,152,775, US 4,794,926 und US 5,035,704 sind Stechhilfen bekannt, die mehrere Lanzetten in sich bevorraten und diese nacheinander einzeln für Stechvorgänge benutzen können. Nach dem Stechvorgang können die Lanzetten einzeln aus dem Gerät entfernt werden. Die Magazinierung und die mögliche automatisierte Bereitstellung der Lanzetten helfen, Fehler beim Einlegen der Lanzette in eine Stechhilfe zu verrneiden. Zur Durchführung der Stechbewegung muss die Lanzette in der Stechhilfe zunächst in eine Stechrichtung bewegt oder ausgelenkt werden und anschließend möglichst schnell in ihre Ausgangslage zurückgeholt werden. Es muss somit sowohl vorwärts gerichtet in Stechrichtung als auch zurück in die Ausgangsposition die Nadel bewegt bzw. die Lanzette bewegt werden. Ein automatischer Lanzettenwechsel ist mit einem derartigen System nur schwer zu realisieren. Zudem weisen derartige Lanzetten einen an den Lanzettenhalter angepassten Lanzettenkörper auf, der eine feste Verbindung zwischen Lanzette und Halter herstellt, um insbesondere die Rückwärtsbewegung sicherzustellen. Daraus ergibt sich, dass derartige Lanzetten oftmals voluminös und ihre Bevorratung in einem Magazin zu großen Dimensionen des gesamten Lanzettensystemes führt.

Aus US 5,578,014 ist ein Lanzettensystem bekannt, bei dem der Antrieb der Lanzette für die Vorwärtsbewegung unabhängig vom Antrieb für die Rückwärtsbewegung arbeitet. Die Vorwärtsbewegung wird durch einen federgetriebenen Stößel bewirkt, der Teil einer Stechhilfe ist und auf die Lanzette von hinten, das heißt von ihrer der Spitze abgewandten Seite, einwirkt. Die Rückwärtsbewegung wird durch eine Feder, die in der Lanzette enthalten ist, angetrieben. Ähnliche Systeme werden in US 5,029,583 und DE-A 198,55,465 beschrieben. Nachteilig ist hierbei, dass jede Lanzette mit einer eigenen Feder ausgerüstet sein muss, was ihre Herstellung kompliziert und teuer macht. Zudem ist eine Miniaturisierung des gesamten Systems nur schwer möglich.

Aus EP 1 190 674 ist ein System zum Bevorraten und Bereitstellen von Lanzetten zur Gewinnung von Körperflüssigkeiten bekannt. Dieses System besteht aus einem Lanzettenmagazin mit einer Mehrzahl von Lanzetten, einem Antriebselement einer Antriebseinheit und einer Rückholvorrichtung, wobei die Rückholvorrichtung mit dem Lanzettenmagazin verbunden und gegenüber diesem verschiebbar ist.

Zugleich ist es unerwünscht, dass die Herstellkosten für derartige Lanzetten, die ja Massenartikel zum Einmalgebrauch sind, hoch sind.

Somit ist es Aufgabe der Erfindung, eine Vorrichtung zum Bevorraten und Bereitstellen von Lanzetten zur Gewinnung von Körperflüssigkeiten zur Verfügung zu stellen, die das Anordnen einer Vielzahl von kostengünstig herstellbaren Lanzetten auf kleinem Raum ermöglicht und einfach in ihrem Gesamtaufbau und somit zuverlässig in ihrer Funktion ist.

Diese Aufgabe wird durch die Merkmale des Patentanspruches 1 gelöst.

Kerngedanke der Erfindung ist es, dass bei einer Vorrichtung zur Bevorratung und Bereitstellen von Lanzetten zur Gewinnung von Körperflüssigkeiten die Vorrichtung Folgendes umfasst:
- Ein Lanzettenmagazin, das eine vorzugsweise im Wesentlichen zylindermantelförmige oder scheibenförmige oder rechteckförmige oder anders geformte Lanzettenbevorratungseinheit mit einer Mehrzahl von im Wesentlichen nadelförmigen Lanzetten und eine Antriebseinheit, weiche eine ausgewählte Lanzette zur Ausführung eines Stechvorganges aus einer Ruheposition in eine Stechposition in Stechrichtung bewegt, umfasst, und
- Eine Rückholeinheit, welche die ausgewählte Lanzette nach der Ausführung des Stechvorganges von der Stechposition in ihre Ruheposition bewegt, wobei die Rückholeinheit mit der Lanzettenbevorratungseinheit verbunden und gegenüber dieser verschiebbar ist und die Antriebseinheit gegenüber der Rückholeinheit in Stechrichtung betrachtet fixiert ist.

Gemäß einer bevorzugten Ausführungsform umfasst die zylindermantelförmige Lanzettenbevorratungseinheit, in der die Lanzetten parallel zueinander auf der Kreisbahn verteilt angeordnet sind und im Wesentlichen sich in Längsachse des Zylindermantels erstrecken, die Rückholeinheit, welche im Wesentlichen zylinderförmig ausgebildet ist. Dieses Umfassen bzw. Umgeben der Rückholeinheit ist zumindest teilweise vorhanden und stellt sicher, dass die Rückholeinheit raumsparend innerhalb des Zylindermantels der Lanzettenbevorratungseinheit angeordnet und verschiebbar sein kann.

Die Antriebseinheit weist zumindest ein stößelartiges Element auf, dessen Stößelende die ausgewählte Lanzette während des Stechvorganges endseitig berührt und innerhalb der Lanzettenbevorratungseinheit in Stechrichtung verschiebt. Hierbei ist das stößelartige Element auch dazu geeignet, die Rückholeinheit gegenüber der Lanzettenbevorratungseinheit zu verschieben. Dies gilt sowohl in Vorwärts- als auch in Rückwärtsrichtung, also während des Stechvorgangs für das Verschieben der Lanzette in die Haut eines menschlichen Körpers hinein und aus diesen wieder heraus.

Jede Lanzette ist innerhalb der zylindermantelartigen Lanzettenbevorratungseinheit in einem separaten Durchgangskanal angeordnet, der sich vorzugsweise in Längsrichtung, also entlang der Längsachse des Zylindermantels der Lanzettenbevorratungseinheit erstreckt. Somit besteht eine Vielzahl von Durchgangskanälen, die auf einer Kreisbahn angeordnet in dem Zylindermantel der Lanzettenbevorratungseinheit, wobei die Nadeln einzeln durch das stößelartige Element angesteuert und verschoben werden können.

Erfindungsgemäß weisen die Lanzetten keine damit fest verbundenen Kunststoffumfassungen auf, sondern jeweils lediglich ein gebogenes Ende, um damit jeweils in eine sich in Radialrichtung erstreckende Vertiefung an der stirnseitigen Oberfläche der teilweise zylinderförmigen Rückholeinheit einzugreifen. Somit kann die Oberfläche und die darin angeordneten Vertiefungen der Rückholeinheit dafür verwendet werden, die Lanzette bei einem Rückholvorgang, also bei einer Bewegung der einzelnen Lanzette in Rückwärtsrichtung, auf schnelle und einfache Art durch das Zurückverschieben der Rückholeinheit wieder aus den menschlichen Körper heraus zu bewegen.

Erfindungsgemäß ist eine erste Clipsverbindung oder eine andere Art von form-, kraft- und/oder stoffschlüssiger Verbindung vorhanden, welche die Antriebseinheit in Form eines stößelartigen Elementes mit der Rückholeinheit, in Stechrichtung betrachtet, fest verbindet. Diese erste Clipsverbindung ist dazu geeignet, das stößelartige Element gegenüber der Rückholeinheit um eine in Stechrichtung verlaufende Längsachse des Lanzettenmagazins zu verdrehen. Somit wird die Clipsverbindung dazu verwendet, die Rückholeinheit in Verschieberichtung bzw. in Stechrichtung sowohl in Vorwärts- als auch in Rückwärtsrichtung während des Stechvorganges fest mit der Antriebseinheit und insbesondere mit dem stößelartigen Element zu verbinden, wobei ein Verdrehen des stößelartigen Elementes gegenüber der Rückholeinheit dazu dient, eine nächste Lanzette für das anschließende Berühren des stößelartigen Elementes mit dem Ende der Lanzette auszuwählen. Eine derartige erste Clipsverbindung ist vorteilhaft zwischen einem Zylinderabschnitt des stößelartigen Elementes und der Innenseite der Rückholeinheit, innerhalb welcher sich der Zylinderabschnitt befindet, angeordnet. Sie besteht aus mindestens einem Vorsprung, der mittels mindestens eines Kunststoffarmes federnd auslenkbar ist und in eine komplementär dazu ausgebildete Rinne eingreift. Die Rinne kann an dem Zylindermantel des stößelartigen Elementes und die Vorsprünge an der Rückholeinheit befestigt sein oder vice versa.

Erfindungsgemäß hält eine zweite Clipsverbindung bei Vorliegen einer Ruheposition sämtlicher Lanzetten, die Rückholeinheit gegenüber der Lanzettenbevorratungseinheit in einer bestimmten Verschiebeposition.

Die Antriebseinheit ist mittels mindestens eines Federelementes federkraftbeaufschlagbar, um das stößelartige Element und die Rückholeinheit in Stechrichtung und entgegengesetzt zu bewegen. Auf diese Weise erübrigen sich Federanordnungen an den einzelnen Lanzetten oder auch an der Rückholeinheit selber. Vielmehr kann die Antriebseinheit die komplette Vorwärts- und Rückwärtsbewegung antreiben, wodurch das Lanzettenmagazin in seiner Gesamtheit ausgewechselt werden kann und mit der Antriebseinheit ein neues Lanzettenmagazin verbunden wird, ohne dass einzelne Antriebselemente innerhalb des Lanzettenmagazins vorliegen müssen. Dies ermöglicht einen einfachen und kostengünstigen Aufbau des Lanzettenmagazins ohne die Anordnung von irgendwelchen Federn oder derartiger Elemente. Auch elektromagnetische Antriebe oder andere kraftbeaufschlagte antriebe sind denkbar.

Weitere vorteilhafte Ausführungsformen ergeben sich aus den nachfolgenden Beschreibungen in Verbindung mit der Zeichnung: hierbei zeigen:
- Fig. 1: in einer perspektivischen Darstellung eine Grundkonstruktion der erfindungsgemäßen Vorrichtung gemäß einer Ausführungsform der Erfindung;
- Fig. 2: in einer Explosionsdarstellung einzelne Teile der erfindungsgemäßen Vorrichtung;
- Fig. 3: in einer Schnittdarstellung das Lanzettenmagazin zusammen mit Teilen der Antriebseinheit der erfindungsgemäßen Vorrichtung;
- Fig. 4: in einer schematischen weiteren Darstellung das Lanzettenmagazin zusammen mit Teilen der Antriebseinheit mit nicht ausgefahrenen Lanzetten gemäß einer Ausführungsform der vorliegenden Erfindung;
- Fig. 5: in einer schematischen weiteren Darstellung das Lanzettenmagazin zusammen mit Teilen der Antriebseinheit mit einer ausgefahrenen Lanzette gemäß einer Ausführungsform der vorliegenden Erfindung;
- Fig. 6: in einer perspektivischen Darstellung das Lanzettenmagazin mit Teilen der Antriebseinheit von schräg oben ohne Außengehäuse;
- Fig. 7: in einer perspektivischen Darstellung Teile des Lanzettenmagazins zusammen mit Teilen derAntriebseinheit in einerAnsicht von schräg unten;
- Fig. 8: in einer perspektivischen Darstellung das Lanzettenmagazin der erfindungsgemäßen Vorrichtung in einer perspektivischen Ansicht von schräg unten,
- Fig. 9: in einer Ansicht von unten das Lanzettenmagazin der erfindungsgemäßen Vorrichtung;
- Fig. 10: in einer Querschnittsdarstellung das Lanzettenmagazin zusammen mit Teilen der Antriebseinheit der erfindungsgemäßen Vorrichtung; und
- Fig. 11: in einer perspektivischen Querschnittsdarstellung Teile der Antriebseinheit der erfindungsgemäßen Vorrichtung.

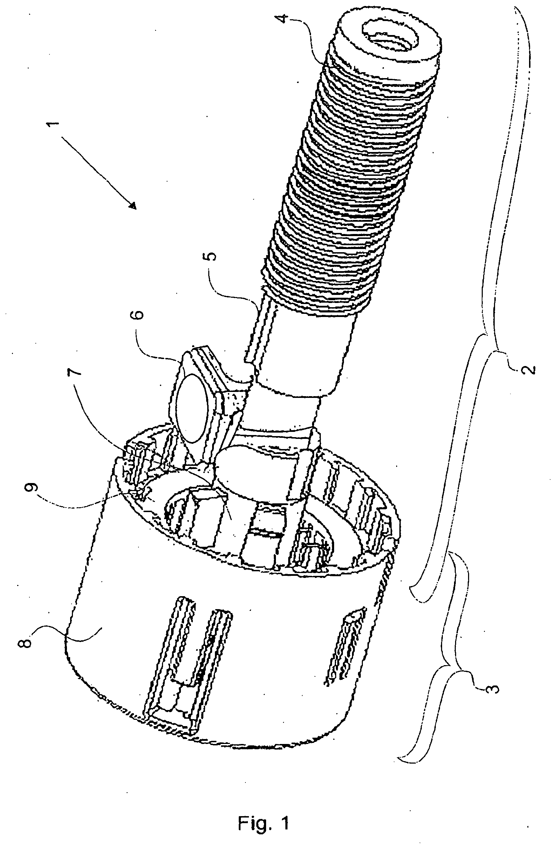

Im Fig. 1 ist in einer perspektivischen Darstellung die gesamte erfindungsgemäße Vorrichtung zum Bevorraten und Bereitstellen von Lanzetten zur Gewinnung von Körperflüssigkeiten ohne weitergehende Verkleidungselemente in ihrer Grundstruktur dargestellt. Diese Vorrichtung 1 weist eine Antriebseinheit 2 und ein Lanzettenmagazin 3 auf, wobei die Antriebseinheit 2 eine Feder, vorzugsweise eine Spiralfeder 4, umfasst, die zum Federkraftbeaufschlagen eines Stößels 7 dient, der sich in Vorwärts- und Rückwärtsrichtung bewegt, wobei die Spiralfeder 4 um einen Zylinder 5 herum angeordnet ist. Eine Auslöseeinheit 6 dient dazu, die Antriebseinheit, insbesondere den Stößel 7 nach vorne, also in Richtung des Lanzettenmagazinausganges am unteren Ende (linke Seite der Abbildung) und anschließend in Rückwärtsrichtung bewegen zu lassen. Diese Auslöseeinheit 6 ist vorzugsweise als Taste angeordnet.

Das Lanzettenmagazin 3 umfasst ein Außengehäuse 8 zusammen mit einer Rückholeinheit 9.

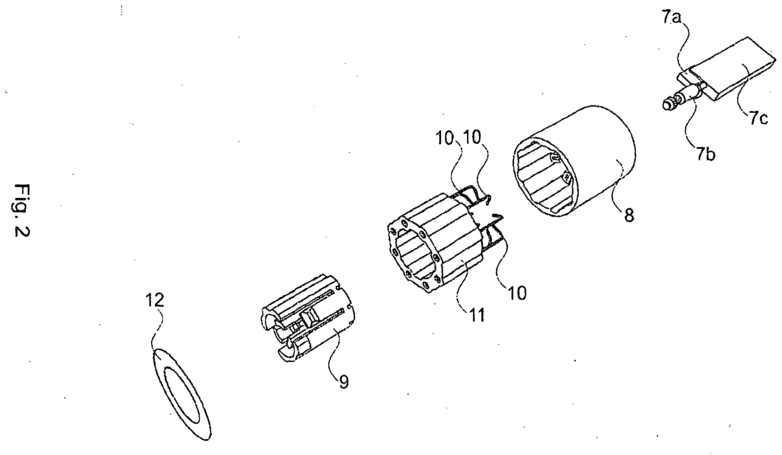

In Fig. 2 sind in einer Explosionsdarstellung Teile der Vorrichtung, insbesondere des Lanzettenmagazins der erfindungsgemäßen Vorrichtung in einer Ausführungsform wiedergegeben. Dieser Darstellung ist zu entnehmen, dass das zylinderförmig aufgebaute Gehäuse die Rückholeinheit 9 und eine zylindermantelförmig aufgebaute Lanzettenbevorratungseinheit 11 umfasst. In dieser Lanzettenbevorratungseinheit 11 ist innerhalb des Mantels eine Mehrzahl an Lanzetten 10 angeordnet, die an ihrem oberen Ende bogenförmig ausgebildet sind, um die Rückholeinheit 9, welche innerhalb der Lanzettenbevorratungseinheit 11 verschiebbar ist, darin eingreifen lassen zu können.

Nach unten hin weist das Lanzettenmagazin eine abschließende Abdeckung 12 auf, die selbstverständlich derart gestaltet ist, dass die Lanzetten nach wie vor aus der Lanzettenbevorratungseinheit 11 nach unten austreten können.

Die Antriebseinheit 2 umfasst den Stößel 7, von dem ein stößelartiges Element 7a auf einzelne Lanzetten in ihrem oberen Endbereich wirken kann und hierdurch ein Verschieben der einzelnen Lanzetten nach unten zum Austritt aus dem Lanzettenmagazin wirkt. Der Stößel 7 weist zudem zur Befestigung eines Grundelementes 7c des Stößels 7 an der Rückholeinheit 9 einen zylinderförmigen Anteil 7b auf.

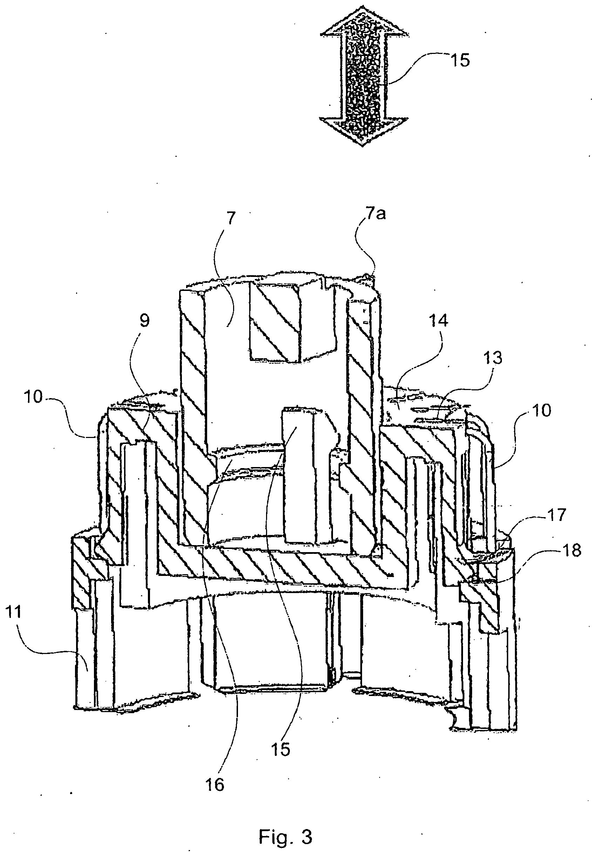

In Fig. 3 wird in einer Schnittdarstellung das Lanzettenmagazin zusammen mit Teilen der Antriebseinheit der erfindungsgemäßen Vorrichtung wiedergegeben. In dieser Darstellung ist deutlich zu sehen, dass der Stößel 7 mittels einer ersten Clipsverbindung 15, 16 mit der Rückholeinheit 9 verbunden ist, wobei der clipsartige Vorsprung 15 an der Rückholeinheit 9 angeordnet ist und eine Rinne oder ein Vorsprung 16 an einer Innenseite des Zylindermantels des Stößels 7 vorliegt. Somit kann mittels der ersten Clipsverbindung der Stößel 7 um seine Längsachse und um die Längsachse der gesamten Vorrichtung und damit auch des Lanzettenmagazins gedreht werden, ist jedoch in Richtung der Längsachse fixiert bzw. fest mit der Rückholeinheit 9 verbunden. Selbstverständlich können derartige clipsartige Vorsprünge, die einzeln oder mehrfach auf einer Kreisbahn angeordnet sein können, auch mit dem Stößel 7 verbunden sein und eine entsprechend komplementär ausgebildete Rinne oder ein kreisförmiger Vorsprung an der Rückholeinheit 9 kann vorliegen.

Die Lanzetten 10 sind derart in den Zylindermantel der Lanzettenbevorratungseinheit 11 angeordnet, dass deren oberes Ende jeweils in eine radial ausgerichtete Vertiefung 13 innerhalb einer Oberfläche 14 der Rückholeinheit 9 eingreift.

Eine zweite Clipsverbindung 17, 18 ist zwischen der Rückholeinheit und der Lanzettenbevorratungseinheit angeordnet, wobei einer oder mehrere clipsartige Vorsprünge 17 mit der Rückholeinheit verbunden ist/sind und ein komplementär ausgebildeter Vorsprung 18 oder eine Rinne, in die der clipsartige Vorsprung 17 eingreifen kann, kreisförmig an der Lanzettenbevorratungseinheit innenseitig vorliegt.

Somit kann mittels der zweiten Clipsverbindung, die dazu geeignet ist, nicht dauerhaft die Rückholeinheit 9 mit der Lanzettenbevorratungseinheit 11 in Längsrichtung der gesamten Vorrichtung zu verbinden, dazu dienen, in einer Ruheposition, in welcher die Rückholeinheit 9 innerhalb der Lanzettenbevorratungseinheit 11 nach oben verschoben ist und keine der Lanzetten am unteren Ende der Lanzettenbevorratungseinhei taustritt, eine mittels Druck zu überwindende Befestigung der Rückholeinheit 9 innerhalb der Lanzettenbevorratungseinheit 11 zu erhalten. Die soll sicherstellen, dass die Rückholeinheit zuverlässig sämtliche Lanzetten innerhalb der Lanzettenbevorratungseinheit 11 hält, solange eine Ruheposition vorliegt. Bei einem Stechvorgang würde dann gemäß dem Doppelpfeil 15 sowohl der Stößel 7 mit dem stößelartigen Element 7a, welches auf eine der Lanzetten wirkt, als auch die Rückholeinheit 9 kurzzeitig in Vorwärtsrichtung nach unten geschoben und in Rückwärtsrichtung nach oben geschoben werden. Dies hat ein schnelles Vor- und Zurückgleiten einer der ausgewählten Lanzetten zur Folge und ermöglicht den Stechvorgang.

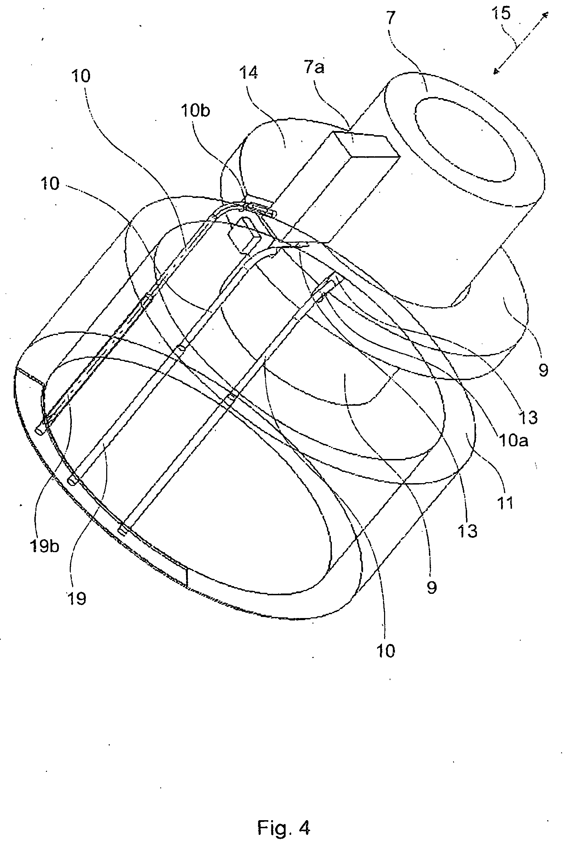

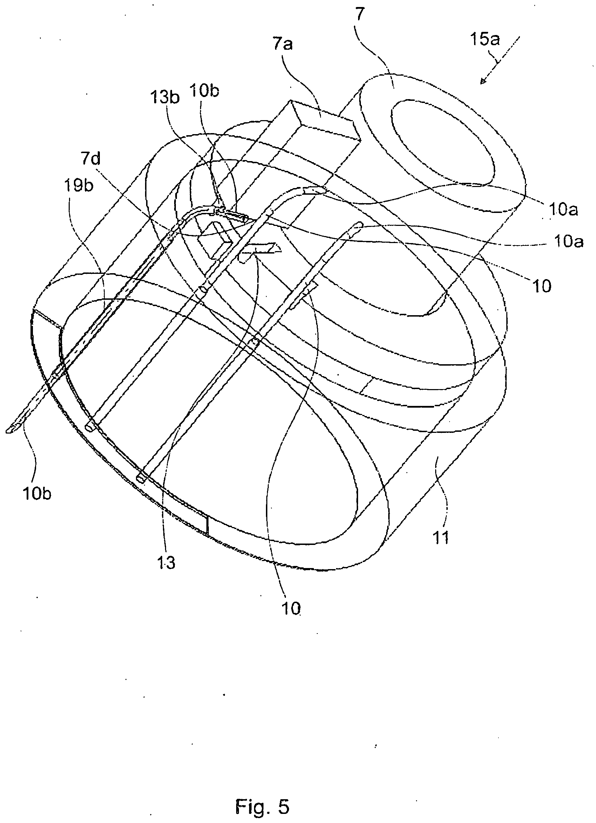

In Fig. 4 ist in einer schematischen weiteren Darstellung das Lanzettenmagazin zusammen mit Teilen der Antriebseinheit der erfindungsgemäßen Vorrichtung wiedergegeben. Dieser Darstellung ist im Vergleich mit der Darstellung gemäß Fig. 5 die Grundfunktion der Vorrichtung zu entnehmen. Durch einen Vergleich zwischen den Darstellungen gemäß Fig. 4 und Fig. 5 wird deutlich, dass das stößelartige Element 7a mit seinem unterseitigen Ende auf eine Lanzette 10b wirkt, die innerhalb einer der Vertiefungen an der Oberfläche 14 der Rückholeinheit 9 angeordnet ist. Diese Lanzette weist an ihrem oberen Ende eine Biegung auf und ergibt somit ebenso wie bei den anderen Lanzetten ein bogenförmiges Ende 10a. Hierdurch wird ein Eingreifen in die Vertiefungen 13 an der Oberfläche 14 ermöglicht.

Es sind in Fig. 4 und Fig. 5 lediglich drei Lanzetten dargestellt, obgleich mehr als drei Lanzetten entlang des Zylindermantels der Lanzettenbevorratungseinheit 11 kreisförmig angeordnet werden können.

Bei einem Nachuntenverschieben gemäß dem Pfeil 15a der Antriebseinheit bzw. des Stößels 7 und damit des stößelartigen Elementes 7a berührt ein unteres Ende 7d das obere Ende 10b der Lanzette 10c und drückt die Lanzette innerhalb eines Durchgangskanales 19b, der für die restlichen Lanzetten 10 ebenso vorhanden ist und mit dem Bezugszeichen 19 gekennzeichnet ist, nach unten, bis am unteren Ende der Lanzettenbevorratungseinheit 11 die Lanzette mit ihrer Spitze austritt. Das obere Ende 10b der Lanzette 10c verbleibt während dieser Vorwärtsbewegung innerhalb einer Vertiefung 13b in der Oberfläche 14 der Rückholeinheit und wird zusammen mit der Rückholeinheit 9 nach unten verschoben Anschließend findet aufgrund einer Federkraftbeaufschlagung der Antriebseinheit 2, die hier nicht näher dargestellt ist, eine Rückwärtsbewegung der gesamten Rückholeinheit zusammen mit der einzelnen Lanzette 10c statt, bis diese wieder an dem unteren Ende der Lanzettenbevorratungseinheit in die Lanzettenbevorratungseinheit an sich eintritt. Die restlichen Lanzetten 10 mit ihren oberen Enden 10a verweilen währenddessen in der vorher vorhandenen Position innerhalb der Lanzettenbevorratungseinheit 11 und werden innerhalb der Durchgangskanäle 19 nicht verschoben. Dies geht deutlich aus Fig. 5 hervor.

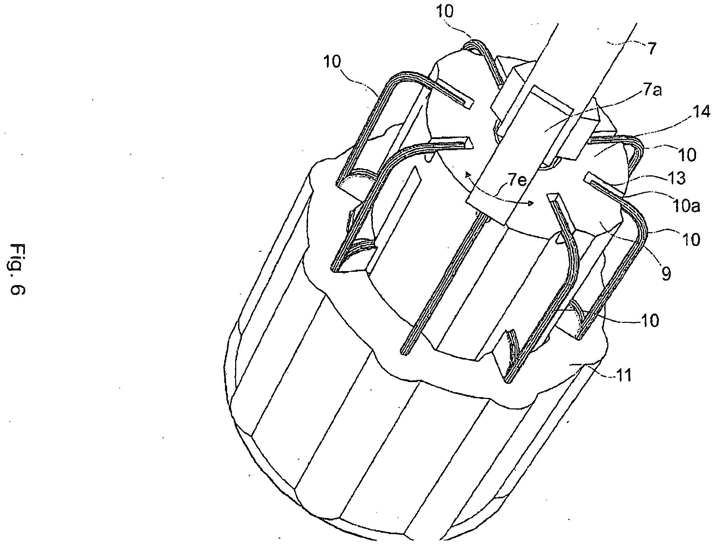

In Fig. 6 ist in einer schematischen Darstellung von schräg oben das Lanzettenmagazin ohne Verkleidungsteile zusammen mit Teilen der Antriebseinheit wiedergegeben. Dieser Darstellung ist zu entnehmen, dass die einzelnen Lanzetten 10 innerhalb der Lanzettenbevorratungseinheit 11 kreisförmig angeordnet sind und mittels der Rückholeinheit 9 in ihrer Position so lange gehalten werden, bis die Rückholeinheit 9 innerhalb der Lanzettenbevorratungseinheit 11 nach unten verschoben wird. Dies geschieht bekannterweise durch das stößelartige Element 7a.

Das stößelartige Element 7a - wie durch den Doppelpfeil 7e dargestellt - kann um eine Längsachse der Vorrichtung und des Lanzettenmagazins gedreht werden, sodass das stößelartige Element 7a mit seiner Unterseite 7d einzelne Lanzetten anfahren kann und somit eine neue Lanzette ausgewählt werden kann.

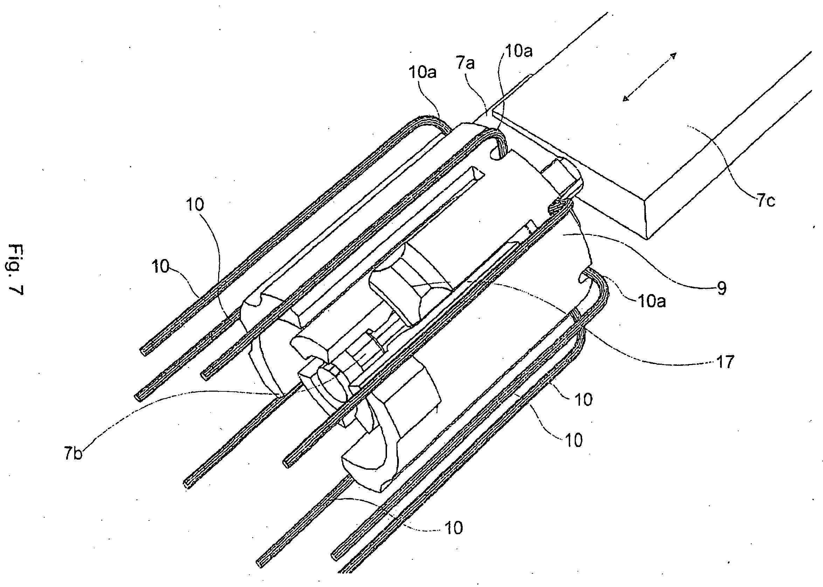

In Fig. 7 sind Teile des Lanzettenmagazins zusammen mit Teilen der Antriebseinheit der erfindungsgemäßen Vorrichtung wiedergegeben. In dieser Darstellung ist die Lanzettenbevorratungseinheit, in welcher die Lanzetten angeordnet sind, nicht wiedergegeben. Vielmehr wird lediglich die Rückholeinheit 9 dargestellt, die mittels einer Clipsverbindung 17 in eine komplementär ausgebildete Rinne oder einen Vorsprung, der hier nicht gezeigt ist, der Lanzettenbevorratungseinheit, die diese Rückholeinheit 9 umfasst bzw. umgibt, eingreift.

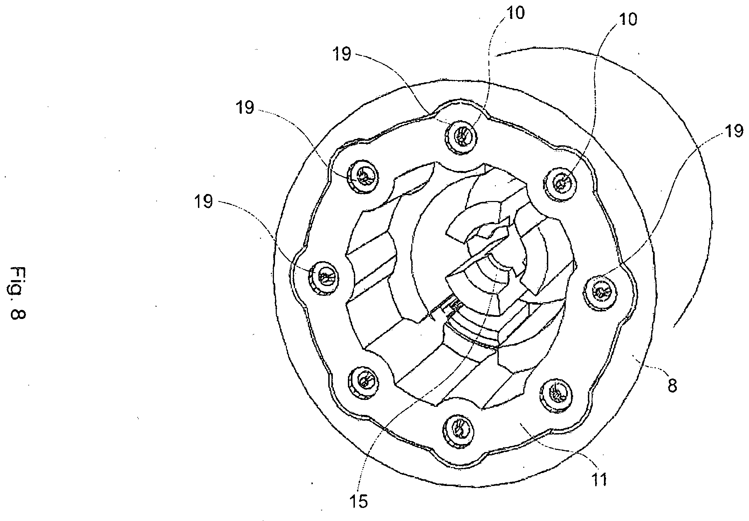

In Fig. 8 ist in einer von unten dargestellten perspektivischen Ansicht das Lanzettenmagazin in der erfindungsgemäßen Vorrichtung wiedergegeben. Dieser Darstellung ist zu entnehmen, dass in Austrittsöffnungen der Durchgangskanäle 19 der einzelnen Lanzettenspitzen der Lanzetten 10 angeordnet sind. Zudem sind von der ersten Clipsverbindung insgesamt drei clipsartige Vorsprünge 15 zu sehen, die in eine hier nicht näher dargestellt Einkerbung, Rinne oder einen Vorsprung des zylinderartigen Anteiles 7b des Stößels 7 eingreifen.

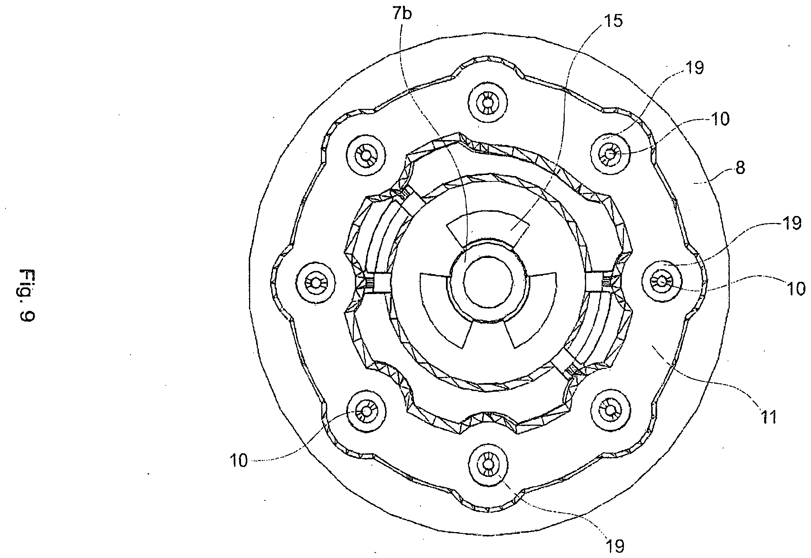

In Fig. 9 ist nochmals in einer Unteransicht das Lanzettenmagazin gemäß Fig. 8 dargestellt. Auch dieser Darstellung ist zu entnehmen, dass clipsartige Vorsprünge 15, die in radialer Richtung nach außen betrachtet nach außen gelenkt werden können, in hier nicht näher dargestellte Rinnen oder Vorsprünge des Zylindermantelanteiles 7b des Stößels 7 eingreifen können.

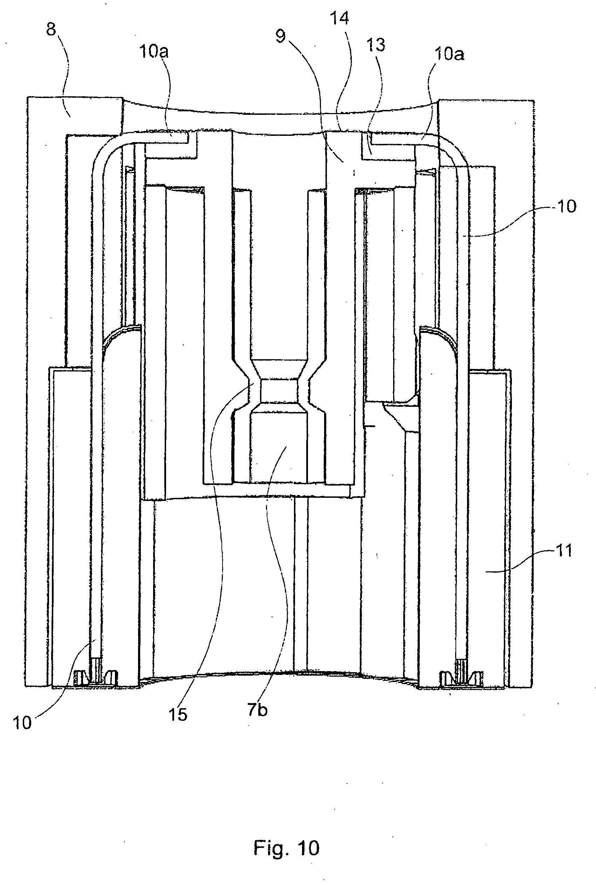

In Fig. 10 ist in einer Schnittdarstellung, in Längsrichtung betrachtet, das Lanzettenmagazin zusammen mit Teilen der Antriebseinheit der erfindungsgemäßen Vorrichtung wiedergegeben. Dieser Darstellung kann deutlich entnommen werden, dass die clipsartigen Vorsprünge 15 der ersten Clipsverbindung in eine kreisförmige Rinne des Teiles 7b des Stößels 7 eingreifen können, um die Lanzettenrückholeinheit 9 dauerhaft mit der Antriebseinheit zumindest ist achsialer Richtung zu verbinden.

Die oberen Enden 10a der Lanzetten 10 sind in gebogener Form in die Vertiefungen 13 der Oberfläche 14 der Rückholeinheit 9 eingreifend angeordnet.

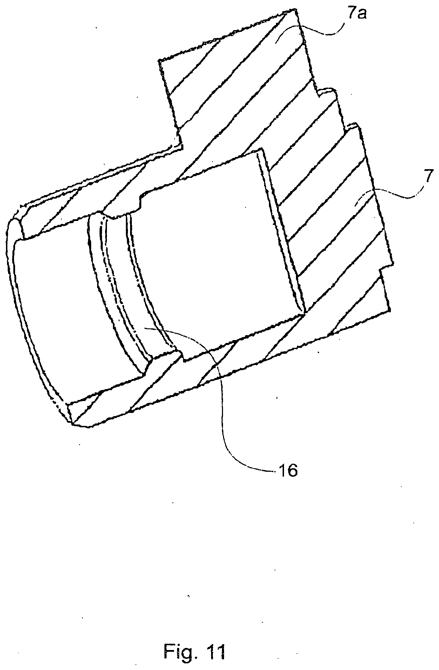

In Fig. 11 ist in einer perspektivischen Querschnittsdarstellung der Stößel in einer möglichen Ausführungsform wiedergegeben. Dieser Darstellung ist zu entnehmen, dass da stößelartige Element 7a außenseitig an einem Zylindermantelteil angeordnet ist. Zugleich ist innerhalb des Zylindermantels eine Rinne in Kombination mit einem Vorsprung 16 angeordnet, die zum Eingreifen der clipsartigen Vorsprünge der Rückholeinheit, welche hier nicht dargestellt sind, dienen.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 2: Antriebseinheit
- 3: Lanzettenmagazin
- 4: Federelement
- 7: Stößel
- 7a: stößelartiges Element
- 7b: Zylindermantelanteil
- 7c: Grundelement
- 7d: Stößelende
- 7e: verdrehte Längsachse des Lanzettenmagazins
- 9: Rückholeinheit
- 10: Lanzetten
- 10a: gebogenes Ende der Lanzetteneinheit
- 10b: ausgewählte Lanzette
- 10c: oberes Ende der Lanzette
- 11: Lanzettenbevorratungseinheit
- 13: Vertiefungen
- 13b: Vorwärtsbewegung innerhalb der Vertiefung
- 14: Oberfläche
- 15: erste Clipsverbindung/Stechrichtung
- 15a: Stechrichtung
- 16: erste Clipsverbindung
- 17: clipsartiger Vorsprung
- 18: ausgebildeter Vorsprung
- 19: Durchgangskanal

## Patentansprüche

1. Vorrichtung zum Bevorraten und Bereitstellen von Lanzetten (10) zur Gewinnung von Körperflüssigkeiten, umfassend:
- Ein Lanzettenmagazin (3), das eine Lanzettenbevorratungseinheit (11) mit einer Mehrzahl von im Wesentlichen nadelförmigen Lanzetten (10) und eine Antriebseinheit (2), welche eine ausgewählte Lanzette (10b) zur Ausführung eines Stechvorgangs aus einer Ruheposition in eine Stechposition in Stechrichtung (15, 15a) bewegt, umfasst, und
- Eine Rückholeinheit (9), welche die ausgewählte Lanzette (10b) nach der Ausführung des Stechvorgangs von der Stechposition in ihre Ruheposition bewegt, wobei die Rückholeinheit (9) mit der Lanzettenbevorratungseinheit (11) verbunden und gegenüber dieser verschiebbar ist,
**dadurch gekennzeichnet, dass**
die Antriebseinheit (2) gegenüber der Rückholeinheit (9) entlang einer Stechrichtung (15, 15a) fixiert ist, wobei eine form- und/oder kraftschlüssige Verbindung vorhanden ist, welche die Antriebseinheit (2) in Form eines stößelartigen Elementes (7a) mit der Rückholeinheit (9), in Stechrichtung betrachtet, fest verbindet, wobei die form-und/oder kraftschlüssige Verbindung eine erste Clipsverbindung (15, 16) ist, wobei eine derartige erste Clipsverbindung (15, 16) zwischen einem Zylinderabschnitt des stößelartigen Elementes (7a) und der Innenseite der Rückholeinheit (9), innerhalb welcher sich der Zylinderabschnitt befindet, angeordnet ist, wobei sie aus mindestens einem Vorsprung besteht, der mittels mindestens eines Kunststoffarmes federnd auslenkbar ist und in eine komplementär dazu ausgebildete Rinne eingreift, wobei jede Lanzette (10) innerhalb der zylindermantelartigen Lanzettenbevorratungseinheit (11) in einem Durchgangskanal (19) angeordnet ist, wobei die Lanzetten (10) jeweils ein gebogenes Ende (10a) aufweisen, um damit in jeweils eine sich in Radialrichtung erstreckende Vertiefung (13) an der stirnseitigen Oberfläche (14) der teilweise zylinderförmigen Rückholeinheit (9) einzugreifen, wobei eine zweite Clipsverbindung (17, 18) bei Vorliegen einer Ruheposition sämtlicher Lanzetten (10) die Rückholeinheit (9) gegenüber der Lanzettenbevorratungseinheit (11) in einer bestimmten Verschiebeposition hält.

2. Vorrichtung nach Anspruch 1,
**dadurch gekennzeichnet, dass**
die Lanzettenbevorratungseinheit (11), welche im Wesentlichen zylindermantelförmig ausgebildet ist und in der die Lanzetten (10) parallel zueinander auf einer Kreisbahn verteilt angeordnet sind, die Rückholeinheit (9), welche im Wesentlichen zylinderförmig ausgebildet ist, zumindest teilweise umfasst, wobei die Rückholeinheit (9) innerhalb der Lanzettenbevorratungseinheit (11) verschiebbar ist.

3. Vorrichtung nach Anspruch 1 oder 2,
**dadurch gekennzeichnet, dass**
das Stößelende (7d) des zumindest einen stößelartigen Elements (7a) die ausgewählte Lanzette (10b) während des Stechvorganges end-seitig berührt und innerhalb der Lanzettenbevorratungseinheit (11) in Stechrichtung (15a) verschiebt.

4. Vorrichtung nach Anspruch 3,
**dadurch gekennzeichnet, dass**
das stößelartige Element (7a) zum Verschieben der Rückholeinheit (9) gegenüber der Lanzettenbevorratungseinheit (11) geeignet ist.

5. Vorrichtung nach einem der vorangegangenen Ansprüche,
**dadurch gekennzeichnet, dass**
die Antriebseinheit (3) mittels mindestens eines Federelementes (4) federkraftbeaufschlagt ist, und das stößelartige Element (7a) und die Rückholeinheit (9) entlang der Stechrichtung (15, 15a) zu bewegen.

## Claims

1. Device for storing and providing lancets (10) for obtaining bodily fluids, comprising:
- a lancet magazine (3), which comprises a lancet storage unit (11), having a plurality of substantially needle-shaped lancets (10), and a drive unit (2) which moves a selected lancet (10b) for carrying out a lancing process from a rest position into a lancing position in a lancing direction (15, 15a), and
- a retraction unit (9), which moves the selected lancet (10b) from the lancing position into the rest position thereof after the lancing process has been carried out, the retraction unit (9) being connected to the lancet storage unit (11) and being displaceable with respect thereto,
**characterised in that**
the drive unit (2) is fixed with respect to the retraction unit (9) along a lancing direction (15, 15a), a positive and/or non-positive connection being present which rigidly connects the drive unit (2) in the form of a plunger-like element (7a) to the retraction unit (9) when viewed in the lancing direction, the positive and/or non-positive connection being a first clip connection (15, 16), a first clip connection (15, 16) of this kind being arranged between a cylinder portion of the plunger-like element (7a) and the inner face of the retraction unit (9), inside which the cylinder portion is located, said first clip connection being composed of at least one projection which can be resiliently deflected by means of at least one plastics arm and engages in a groove designed complementarily thereto, each lancet (10) being arranged inside the cylindrical shell-like lancet storage unit (11) in a through-duct (19), the lancets (10) each comprising a curved end (10a) so as to each engage therewith in a depression (13), extending in a radial direction, on the end surface (14) of the retraction unit (9), which is cylindrical at least in part, a second clip connection (17, 18) holding the retraction unit (9) in a particular displacement position with respect to the lancet storage unit (11) if all of the lancets (10) are in a rest position.

2. Device according to claim 1,
**characterised in that**
the lancet storage unit (11), which is in a substantially cylindrical shell form and in which the lancets (10) are distributed mutually parallel on a circular path, encloses the retraction unit (9), which is substantially cylindrical, at least in part, the retraction unit (9) being displaceable inside the lancet storage unit (11).

3. Device according to claim 1 or claim 2,
**characterised in that**
the plunger end (7d) of the at least one plunger-like element (7a) touches an end of the selected lancet (10b) during the lancing process and displaces the selected lancet in the lancing direction (15a) inside the lancet storage unit (11).

4. Device according to claim 3,
**characterised in that**
the plunger-like element (7a) is suitable for displacing the retraction unit (9) with respect to the lancet storage unit (11).

5. Device according to any of the preceding claims,
**characterised in that**
the drive unit (3) is loaded with spring force by means of at least one spring element (4) so as to move the plunger-like element (7a) and the retraction unit (9) along the lancing direction (15, 15a).

## Revendications

1. Dispositif de stockage et de disposition de lancettes (10) pour le prélèvement de fluides corporels, comprenant:
- un chargeur à lancettes (3), qui comprend une unité de stockage de lancettes (11) avec une pluralité de lancettes (10) essentiellement en forme d'aiguilles et une unité d'entraînement (2), qui déplace une lancette (10b) sélectionnée pour la réalisation d'un processus de piquage d'une position de repos vers une position de piquage dans la direction de piquage (15, 15a), et
- une unité de rappel (9) qui déplace la lancette (10b) sélectionnée, après la réalisation du processus de piquage, de la position de piquage vers sa position de repos, l'unité de rappel (9) étant reliée à l'unité de stockage de lancettes (11) et étant coulissante par rapport à celle-ci,
**caractérisé en ce que**
l'unité d'entraînement (2) est fixée par rapport à l'unité de rappel (9) le long d'une direction de piquage (15, 15a), une liaison par coopération de formes et/ou par friction étant présente, laquelle relie fermement l'unité d'entraînement (2), sous forme d'un élément de type poussoir (7a), à l'unité de rappel (9), vue dans la direction de piquage, la liaison par coopération de formes et/ou par friction étant une première liaison par clips (15, 16), une telle première liaison par clips (15, 16) étant disposée entre une partie cylindrique de l'élément de type poussoir (7a) et le côté intérieur de l'unité de rappel (9), à l'intérieur de laquelle se trouve la partie cylindrique, et comprenant au moins une saillie, qui est apte à être déviée de façon élastique au moyen d'au moins un bras en matière plastique et à s'engager dans une rainure configurée pour lui être complémentaire, chaque lancette (10) se trouvant à l'intérieur de l'unité de stockage de lancettes (11), en forme d'enveloppe externe cylindrique, dans un canal de passage (19), les lancettes (10) présentant à chaque fois une extrémité recourbée (10a) afin de s'emboîter à chaque fois dans un évidement (13) s'étendant dans la direction radiale au niveau de la surface frontale (14) de l'unité de rappel (9) au moins partiellement cylindrique, une deuxième liaison par clips (17, 18), lors de l'existence d'une position de repos de l'ensemble des lancettes (10), maintenant l'unité de rappel (9) dans une position de coulissement déterminée par rapport à l'unité de stockage de lancettes (11).

2. Dispositif selon la revendication 1,
**caractérisé en ce que**
l'unité de stockage de lancettes (11), qui est conçue globalement sous la forme d'une enveloppe externe cylindrique et dans laquelle les lancettes (10) sont parallèles entre elles et réparties sur une piste circulaire, entoure au moins partiellement l'unité de rappel (9), qui présente essentiellement une forme cylindrique, l'unité de rappel (9) étant coulissante à l'intérieur de l'unité de stockage de lancettes (11).

3. Dispositif selon l'une des revendications 1 ou 2,
**caractérisé en ce que**
l'extrémité de poussoir (7d) dudit au moins au moins un élément de type poussoir (7a) est en contact, à son extrémité, avec la lancette (10b) sélectionnée pendant le processus de piquage et la pousse à l'intérieur de l'unité de stockage de lancettes (11) dans la direction de piquage (15a).

4. Dispositif selon la revendication 3,
**caractérisé en ce que**
l'élément en forme de poussoir (7a) est conçu pour faire coulisser l'unité de rappel (9) par rapport à l'unité de stockage de lancettes (11).

5. Dispositif selon l'une des revendications précédentes,
**caractérisé en ce que**
l'unité d'entraînement (3) est contrainte par une force élastique à l'aide d'au moins un élément formant ressort (4) pour déplacer l'élément de type poussoir (7a) et l'unité de rappel (9) le long de la direction de piquage (15, 15a).
